# EUROPÄISCHE PATENTSCHRIFT

(11) **EP 2 153 784 B1**
(45) Veröffentlichungstag und Bekanntmachung des Hinweises auf die Patenterteilung: **18.01.2017**
(21) Anmeldenummer: 09405128.1
(22) Anmeldetag: 27.07.2009
(51) Int. Cl.: A61B 17/70

(54) **Stabilisierungsanordnung der Wirbelsäule mit wenigstens einem Verbindungsstab**
Spinal stabilisation assembly with at least one connecting rod
Agencement de stabilisation spinal doté d'au moins une barre de jonction

(30) Priorität: 11.08.2008 CH 12472008
(43) Veröffentlichungstag der Anmeldung: 17.02.2010
(73) Patentinhaber: Sepitec Foundation, 9490 Vaduz (LI)
(72) Erfinder: Fiechter, Meinrad, 3110 Münsingen (CH); Magerl, Friedrich, 9011 St. Gallen (CH); Wieling, Ronald, 9453 Eichberg (CH)
(74) Vertreter: Rutz, Andrea

(56) Entgegenhaltungen:
- EP-A- 0 346 521
- EP-A- 1 808 141
- EP-A- 1 857 065
- WO-A-2005/041795
- WO-A-2007/027934
- US-A- 5 129 388
- US-A1- 2008 086 130

## Beschreibung

Die Erfindung betrifft eine Stabilisierungsanordnung nach dem Oberbegriff des Anspruchs 1.

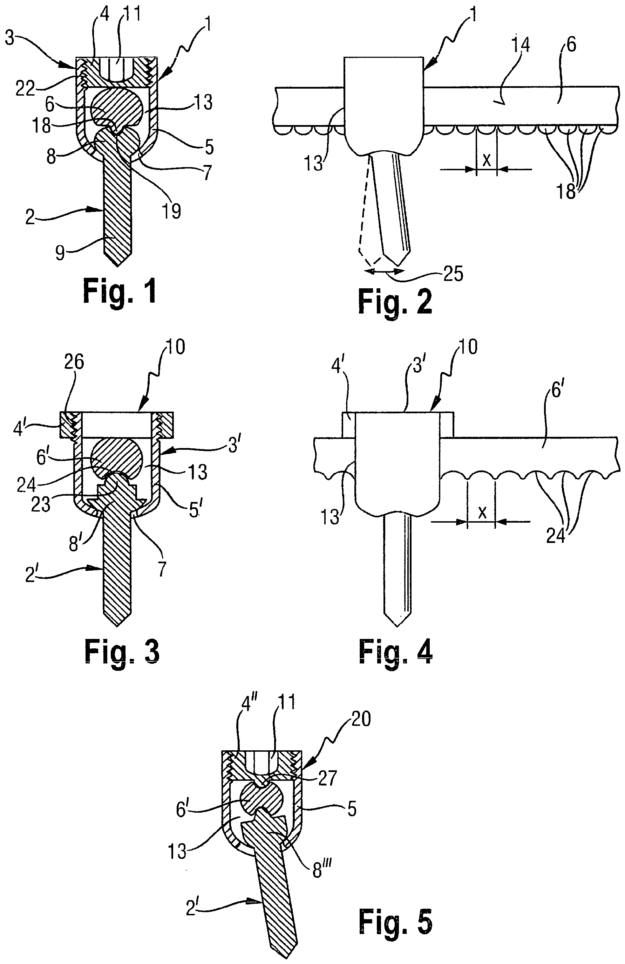

Eine Stabilisierungsanordnung dieser Art ist im Stand der Technik durch die US 2008/0086130 A bekannt geworden. Der Verbindungsstab besitzt einen unrunden Querschnitt und kann dadurch in Umfangsrichtung mit dem Klemmelement formschlüssig fixiert werden. Bei einem Verbindungsstab mit rundem Querschnitt sind Rippen vorgesehen, die sich in Längsrichtung des Verbindungsstabs erstrecken und die an korrespondierende Rippen der Klemmvorrichtung anlegbar sind (Fig. 3).

Die EP 1 808 141 offenbart eine Stabilisierungsanordnung, bei welcher der Verbindungsstab in Umfangsrichtung verlaufende Rillen aufweist. Eine Klemmschraube besitzt entsprechende Vorsprünge, wodurch eine in Längsrichtung formschlüssige Fixierung des Verbindungsstabs an der Klemmschraube erreicht werden kann.

Eine weitere Stabilisierungsanordnung ist im Stand der Technik beispielsweise durch die US 5,474,555 bekannt geworden. Dieser ist ein Rundstab und verbindet wenigstens zwei Pedikelschrauben miteinander. Diese Pedikelschrauben sind jeweils polyaxial mit einem hülsenförmigen Träger verbunden, in welchem der Verbindungsstab reibschlüssig fixiert ist. Zum Fixieren des Verbindungsstabes ist als Klemmelement eine Mutter vorgesehen, welche auf den Träger aufgeschraubt ist und den Verbindungsstab festklemmt. Durch die Kompression der Mutter wird der Verbindungsstab reibschlüssig im Träger fixiert und damit mit der entsprechenden Pedikelschraube fest verbunden. Mit einem solchen Verbindungsstab bzw. einer entsprechenden Stabilisierungsanordnung können benachbarte Wirbel stabilisiert werden. Wesentlich ist die Stabilität der Verbindung. Relative Verschiebungen zwischen dem Verbindungsstab und den Pedikelschrauben müssen auch bei längerer Verwendung vermieden werden. Damit dies gewährleistet ist, ist ein entsprechend hoher Kompressionsdruck erforderlich. Bekannte Verbindungsstäbe werden aus Titan, Implantatenstahl, faserverstärkte Kunststoffe oder anderen biokompatiblen Materialien hergestellt.

Die WO 2005/084566 A1 offenbart einen Verbindungsstab für Knochenverbindungselemente, der einen in Richtung der Längsachse Hohlraum sowie radial durchdringende Längsschlitze aufweist. Die Längsschlitze sollen die Elastizität des Verbindungsstabs so erhöhen, dass diesen dadurch ein gebogenes Rohr implantierbar ist.

Die WO 2007/027934 und die EP-A-1 857 065 offenbaren jeweils eine Stabilisierungsanordnung mit einem Verbindungsstab, der an einer Aussenseite eine Mehrzahl von Längsrillen aufweist, die eine Drehung in Umfangsrichtung verhindern sollen. Die WO 2005/041795 offenbart einen Verbindungsstab der im Querschnitt etwa oval ausgebildet ist und eine Vielzahl von Vertiefungen aufweist, die eine Verschiebung des Verbindungsstabs in dessen Längsrichtung verbinden sollen.

US5,129,388 offenbart eine Stabilisierungsanordnung nach Anspruch 1, nur ist hier keine formschlüssige Fixierung in Umfangsrichtung vorgesehen.

Der Erfindung liegt die Aufgabe zu Grunde, eine Stabilisierungsanordnung der genannten Art zu schaffen, die unabhängig vom Werkstoff eine wesentlich höhere Stabilität der Verbindung ermöglicht und die trotzdem vergleichsweise kostengünstig hergestellt werden kann.

Die Aufgabe ist erfindungsgemäss bei einer gattungsgemässen Stabilisierungsanordnung gemäss Anspruch 1 gelöst. Die erfindungsgemässe Stabilisierungsanordnung ermöglicht somit formschlüssige Verbindungen zu den Klemmorganen. Die Klemmorgane können jeweils mit einer Pedikelschraube oder auch mit einem anderen Veranckungselement mit einem Wirbelkörper verbunden werden. Der Verbindungsstab kann so montiert werden, dass er in Längsrichtung der Wirbelsäule verläuft, denkbar ist aber auch eine Anordnung, bei welcher der Verbindungsstab eine transversale Verbindung zwischen zwei längs verlaufenden Verbindungsstäben bildet. Wird das Klemmorgan mit einer Pedikelschraube verbunden, so kann diese Schraube polyaxial oder auch monoaxial winkelstabil sein. Im Querschnitt ist der Verbindungsstab im wesentlichen kreisförmig. Es ist aber auch unrunder und insbesondere ein ovaler, sternförmiger oder beispielsweise halbmondförmiger Querschnitt denkbar.

Nach einer Weiterbildung der Erfindung ist vorgesehen, dass im wesentlichen die gesamte Aussenseite der Verbindungsstäbe mit Vertiefungen oder Erhöhungen versehen ist. Denkbar ist aber auch eine Ausführung, bei welcher lediglich ein Teilbereich der Aussenseite mit Vertiefungen oder Erhöhung versehen sind. Gemäss der Erfindung sind konkave Vertiefungen oder konvexe Erhöhungen vorgesehen.

Nach der Erfindung sind die Vertiefungen und Erhöhungen so ausgebildet, dass der Formschluss in Längsrichtung als auch in Umfangsrichtung gewährleistet ist. Die Vertiefungen sind hierbei vorzugsweise konkave halbkugelförmige Vertiefungen bzw. konvexe halbkugelförmige Erhöhungen. Damit eine stabile mechanische Verbindung möglich ist, ist gemäss einer Weiterbildung der Erfindung vorgesehen, dass der Abstand zwischen zwei benachbarten Vertiefungen bzw. Erhöhungen grösser als 0,5 mm ist. Am Klemmorgan sind korrespondierende Vertiefungen bzw. Erhöhrungen vorgesehen, welche wenigstens mit einer Vertiefung bzw. Erhöhung des Verbindungsstabes zusammenwirken. Diese Vertiefungen oder Erhöhungen des Klemmorgans können an einem Verankerungsteil, insbesondere und beispielsweise an einer Pedikelschraube oder/und an einem Klemmelement angeordnet sein. Beispielsweise kann eine Pedikelschraube an ihrem Kopf eine kugelförmige Erhöhung oder eine entsprechende Vertiefung aufweisen. Eine Erhöhung oder Vertiefung kann beispielsweise auch an einer Klemm- oder Spannmutter angeordnet sein. Da der Verbindungsstab eine Vielzahl von Vertiefungen und Erhöhungen aufweist, kann dieser in jeder gewünschten Position geklemmt werden.

Die erfindungsgemässe Stabilisierungsanordnung weist zwei Klemmvorrichtungen auf, die jeweils eine Durchgangsöffnung aufweisen, durch welche der Verbindungsstab hindurchschiebbar ist und in welcher er in Längsrichtung und Umfangsrichtung formschlüssig fixierbar ist. Wesentlich ist somit auch hier die formschlüssige Verbindung in Längsrichtung und Umfangsrichtung des Verbindungsstabes. Die Stabilisierungsanordnung kann zur Verankerung in Wirbelkörpern Pedikelschrauben aufweisen, dies ist aber wie erwähnt nicht zwingend, da auch andere Verankerungsmittel möglich sind. Dies Stabilisierungsanordnung kann auch transversale Verbindungsstäbe aufweisen. Eine formschlüssige Verbindung ist sowohl bei transversalen Verbindungsstäben als auch Verbindungsstäben in Längsrichtung der Wirbelsäule denkbar.

Weitere vorteilhafte Merkmale ergeben sich aus den abhängigen Patentansprüchen, der nachfolgenden Beschreibung sowie der Zeichnung.

Ausführungsbeispiele der Erfindung werden nachfolgende anhand der Zeichnung näher erläutert. Es zeigen:
- Fig. 1: ein Schnitt durch eine erfindungsgemässe Stabilisierungsanordnung,
- Fig. 2: eine Teilansicht einer erfindungsgemässen Verbindungsanordnung,
- Fig. 3: ein Schnitt durch eine Variante einer erfindungsgemässen Stabilisierungsanordnung,
- Fig. 4: eine Teilansicht der Stabilisierungsanordnung gemäss Fig. 3,
- Fig. 5: ein Schnitt durch eine erfindungsgemässe Stabilisierungsanordnung gemäss einer weiteren Variante,
- Fig. 6a, 6b: Ansichten einer Pedikelschraube,
- Fig. 7a, 7b: Ansichten einer Pedikelschraube gemäss einer Variante,
- Fig. 8: ein Schnitt durch ein Klemmelement,
- Fig. 9, 10: Ansichten erfindungsgemässer Verbindungsstäbe und
- Fig. 11a und 11b: räumlich Ansichten von Pedikelschrauben.

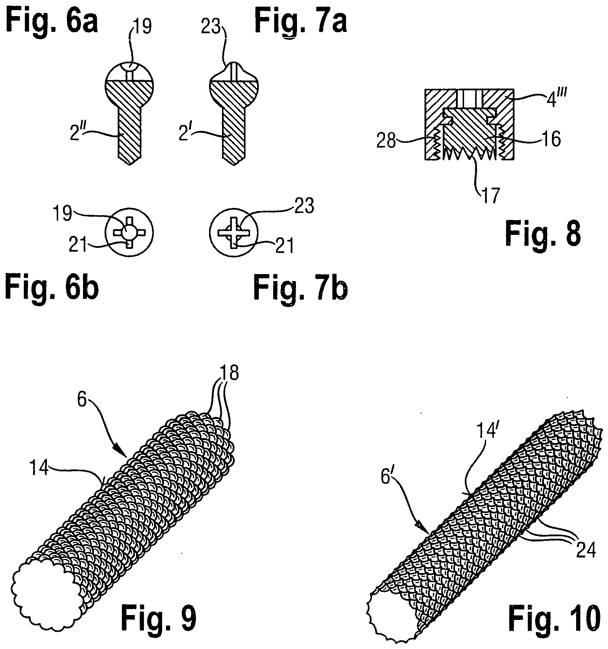

Die in den Figuren 1 und 2 gezeigte Stabilisierungsanordnung 1 besitzt ein Klemmvorrichtung 3, die eine Pedikelschraube 2 mit einem Verbindungsstab 6 verbindet. Die Pedikelschraube 2 besitzt einen Schaft 9 und einen Schraubenkopf 8, der eine Vertiefung 19 sowie gemäss den Figuren 6a und 6b Schlitze 21 aufweist. Die Schlitze 21 dienen für den Ansatz eines hier nicht gezeigten Werkzeuges, mit dem die Pedikelschraube 2 in einen hier nicht gezeigten Wirbelkörper eingeschraubt werden kann. Der Kopf 8 liegt am Rand einer Öffnung 7 eines hülsenförmigen Trägers 5 auf. Die Pedikelschraube 2 ist so im Träger 5 gelagert, dass ihre Achse gemäss Doppelpfeil 25 der Figur 2 polyaxial veränderbar ist. Grundsätzlich kann die Pedikelschraube 2 hier aber auch monoaxial mit dem Träger 5 verbunden sein. Polyaxialität ist hier somit nicht zwingend.

Die Klemmvorrichtung 3 besitzt zudem ein Klemmelement 4, das in ein Innengewinde 22 des Trägers 5 eingeschraubt ist. Für den Ansatz eines Werkzeuges besitzt das Klemmelement 4 beispielsweise einen Innensechskant 11. Durch Anziehen des Klemmelementes 4 kann der Verbindungsstab 6 an die Pedikelschraube 2 angepresst werden. Dadurch wird wiederum der Kopf 8 der Pedikelschraube 2 an den Träger 5 angepresst und dadurch geklemmt.

Der Verbindungsstab 6 ist ein Rundstab und besitzt gemäss Figur 9 eine Aussenseite 14 mit einer Mehrzahl von Erhöhungen 18. Vorzugsweise ist die gesamte Aussenseite 14 mit solchen Erhöhungen 18 versehen. Es ist jedoch auch denkbar, dass lediglich ein Teilbereich der Aussenseite 14 mit solchen Erhöhungen 18 versehen ist. Diese Erhöhungen 18 sind beispielsweise kuppelförmige Erhöhungen mit einer konvexen Aussenseite. Der in Figur 2 gezeigt Abstand x zwischen zwei Erhöhungen 18 ist vorzugsweise grösser als 0,5 mm, vorzugsweise grösser als 0,7 mm und noch bevorzugter grösser als 0,9 mm. Der Abstand x ist vorzugsweise kleiner als 1 mm, vorzugsweise kleiner als 0,8 mm und noch bevorzugter kleiner als 0,7 mm. Die Erhöhungen 18 sind korrespondierend zur Vertiefung 24 ausgebildet. Eine Vertiefung 19 kann somit eine Erhöhung 18 aufnehmen. Die Vertiefung 19 kann jedoch auch so ausgebildet sein, dass sie mehr als eine Erhöhung 18 aufnehmen kann. Die Vertiefung 19 ist zudem so ausgebildet, dass sie in jeder Winkelstellung der Pedikelschraube 2 mit wenigstens einer Erhöhung 18 des Verbindungsstabes 6 in Eingriff bringbar ist. Die Pedikelschraube 2 kann somit auch zwei oder mehr als zwei Vertiefungen 19 oder weiter unten erwähnte Erhöhungen 23 aufweisen.

Die Vertiefung 19 und die mit dieser in Eingriff befindlichen Erhöhung 18 ergeben eine formschlüssige Verbindung zwischen der Pedikelschraube 2 und dem Verbindungsstab 6. Ist das Klemmelement 4 angezogen, so kann sich der Verbindungsstab 6 auch bei vergleichsweise grosser Belastung bezüglich der Klemmvorrichtung 3 und somit bezüglich der Pedikelschraube 2 oder einem anderen Verankerungselement nicht bewegen. Dies auch bei dauernder und/oder wiederholender Belastung in Längsrichtung und Umfangsrichtung des Verbindungsstabes 6.

Der Verbindungsstab 6 kann an sich aus einem beliebigen biokompatiblen Werkstoff hergestellt werden. Geeignete Werkstoffe sind insbesondere Titan, Implantatenstahl, Kunststoffe oder faserverstärkte Kunststoffe. Die Erhöhungen 18 können mechanisch beispielsweise durch Fräsen oder durch Umformen hergestellt werden. Der Verbindungsstab 6 kann gerade oder auch gekrümmt sein. Er besitzt in jedem Fall zwei Enden und kann seitlich oder von oben in einen Durchgang 13 des Trägers 5 eingesetzt werden.

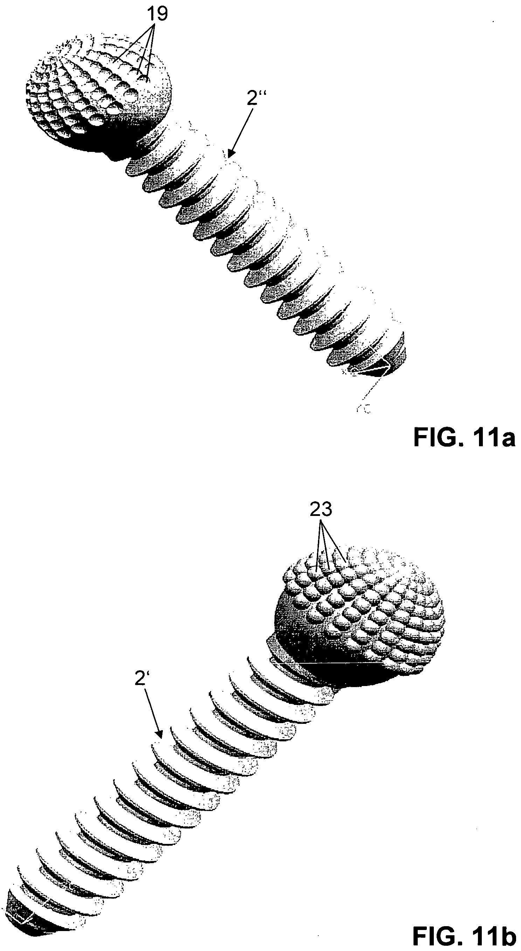

Die Figuren 3 und 4 zeigen eine Stabilisierungsanordnung 10, die eine Klemmvorrichtung 3', ein Klemmelement 4' und einen Träger 5' aufweist. Das Klemmelement 4' ist in diesem Fall auf ein Aussengewinde 26 des Trägers 5' aufgeschraubt. Die Pedikelschraube 2' besitzt einen Kopf 8' der eine kugelförmige Erhöhung 23 besitzt. Die Pedikelschraube 2' ist mit einem Verbindungsstab 6' in Eingriff, der gemäss Figur 10 eine Aussenseite 14' mit einer Vielzahl von Vertiefungen 24 aufweist. Die Vertiefungen 24 entsprechen funktionell den Erhöhungen 18. Sie dienen somit ebenfalls zum Herstellen einer formschlüssigen Verbindung mit der Pedikelschraube 2' oder einem anderen geeigneten Verankerungselement. Die Vertiefungen 24 sind vorzugsweise korrespondierend zur Erhöhung 23 ausgebildet. Die Erhöhung 23 kann hier aber auch so ausgebildet sein, dass sie gleichzeitig mit zwei oder mehr als zwei Vertiefungen 24 in Eingriff bringbar ist. In der Figur 3 ist aus zeichnerischen Gründen lediglich eine der Vertiefungen 24 gezeigt. Auch in diesem Fall sind die Vertiefungen 24 und die Erhöhung 23 so ausgebildet, dass die Pedikelschraube 2' in jeder Winkelstellung formschlüssig mit dem Verbindungsstab 6' verbindbar ist. Die Figuren 11a und 11b zeigen die Pedikelschrauben 2" und 2', die wie ersichtlich jeweils einen Kopf mit einer Vielzahl von Vertiefungen 24 bzw. Erhöhungen 23 aufweisen.

Die Figur 5 zeigt eine Stabilisierungsanordnung 20, die sich von den Stabilisierungsanordnung 1 und 10 im wesentlichen dadurch unterscheidet, dass ein Klemmelement 4" vorgesehen ist, das mit dem Verbindungsstab 6' ebenfalls formschlüssig verbunden ist. Hierzu besitzt das Klemmelement 4" an einer Unterseite wenigstens eine Erhöhung 27, die korrespondierend zu einer Vertiefung 24 ausgebildet ist. Bei der Stabilisierungsanordnung 20 ist der Verbindungsstab 6' somit durch eine formschlüssige Verbindung zur Pedikelschraube 2' als auch mit einer formschlüssigen Verbindung mit dem Klemmelement 4" verbunden. Dadurch ergibt sich eine besonders stabile Fixierung. Auch in diesem Fall sind aus zeichnerischen Gründen nicht alle Vertiefungen 24 gezeigt.

Wie die Figuren 6a, 6b, 7a und 7b zeigen, können im Bereich der Vertiefung 19 bzw. der Erhöhung 23 Schlitze 21 vorgesehen sein, die ein Drehen der Pedikelschraube 2" bzw. 2' mit einem geeigneten Instrument ermöglichen. Anstelle der Schlitze 21 sind auch andere Formen des Angriffs, beispielsweise Innen- oder Aussensechskante möglich

Die Figur 8 zeigt eine Variante eines Klemmelementes 4"'. Dieses besitzt einen zylindrischen und frei drehbaren Einsatz 16, der an einer Unterseite eine Verzahnung 17 besitzt. Diese Verzahnung 17 wird beim Anziehen des Klemmelementes 4"' in die Aussenseite des Verbindungsstabes 6 bzw. 6' gepresst. Dies ist möglich, da der Einsatz 16 im Klemmelement 4 frei drehbar ist. Das Klemmelement 4"' ist mit einem Innengewinde 28 versehen, mit dem es auf den Träger 5' aufgeschraubt ist. Das Gewinde 28 kann eine Sägezahnform oder eine Trapezgewindeform aufweisen, wodurch ein unbeabsichtigtes Lösen des Klemmelementes 4"' verhindert werden kann.

**Bezugszeichenliste**

| | | | |
|---|---|---|---|
| 1 | Stabilisierungsanordnung | 15 | Hülse |
| 2 | Pedikelschraube | 16 | Einsatz |
| 3 | Klemmvorrichtung | 17 | Verzahnung |
| 4 | Klemmelement | 18 | Erhöhung |
| 5 | Träger | 19 | Vertiefung |
| 6 | Verbindungsstab | 20 | Stabilisierungsanordnung |
| 7 | Öffnung | 21 | Schlitz |
| 8 | Schraubenkopf | 22 | Innengewinde |
| 9 | Schaft | 23 | Erhöhung |
| 10 | Stabilisierungsanordnung | 24 | Vertiefung |
| 11 | Innensechskant | 25 | Doppelpfeil |
| 12 | Aussenseite | 26 | Aussengewinde |
| 13 | Durchgang | 27 | Erhöhung |
| 14 | Aussenseite | 28 | Gewinde |

## Patentansprüche

1. Stabilisierungsanordnung mit
einem Verbindungsstab und
wenigstens zwei Klemmvorrichtungen (3), die jeweils mit einem Wirbelkörper einer Wirbelsäule verbindbar sind, und die jeweils einen Träger (5) mit einem Innengewinde (22) oder einem Aussengewinde (26) aufweisen und mit einer Pedikularschraube (2) verbunden sind,
wobei der Verbindungsstab ein erstes und ein zweites Ende sowie eine sich zwischen diesen Enden erstreckende Aussenseite (14) aufweist, an die jeweils ein Klemmelement (4) der genannten Klemmvorrichtungen (3) anlegbar ist, wobei die genannte Aussenseite (14) des Verbindungsstabes mit einer Vielzahl von konkaven Vertiefungen (24) oder konvexen Erhöhungen (18) zum Bilden einer formschlüssigen Verbindung in Umfangsrichtung und in Längsrichtung versehen ist,
wobei die beiden Klemmvorrichtungen (3) jeweils eine Durchgangsöffnung (34) aufweisen, durch welche der Verbindungsstab (6) hindurchschiebbar ist und in welcher er in Längsrichtung und in Umfangsrichtung formschlüssig fixierbar ist,
wobei das Klemmelement (4) der Klemmvorrichtungen (3) jeweils in das Innengewinde (22) des Trägers (5) einschraubbar bzw. auf das Aussengewinde (26) des Trägers (5) aufschraubbar ist,
und wobei das Klemmelement (4) wenigstens eine Erhöhung (27) oder wenigstens eine Vertiefung aufweist, die für eine formschlüssige Verbindung an die wenigstens eine konkave Vertiefung (24) bzw. an die wenigstens eine konvexe Erhöhung (18) des Verbindungsstabs anlegbar ist, so dass bei angezogenem Klemmelement (4) der Verbindungsstab sowohl in Längsrichtung als auch in Umfangsrichtung formschlüssig in der Klemmvorrichtung fixiert ist, und/oder die Pedikularschraube (2) eine Erhöhung (23) oder eine Vertiefung (19) aufweist, die jeweils zum Bilden einer formschlüssigen Verbindung an die wenigstens eine konkave Vertiefung (24) bzw. konvexe Erhöhung (18) des Verbindungsstabs (6) anlegbar ist, so dass bei angezogenem Klemmelement (4) der Verbindungsstab sowohl in Längsrichtung als auch in Umfangsrichtung formschlüssig in der Klemmvorrichtung fixiert ist.

2. Stabilisierungsanordnung nach Anspruch 1, **dadurch gekennzeichnet, dass** er einen im wesentlichen runden, ovalen, halbmondförmigen oder sternförmigen Querschnitt aufweist.

3. Stabilisierungsanordnung nach Anspruch 1 oder 2, **dadurch gekennzeichnet, dass** im wesentlichen die gesamte Aussenseite (14) mit Vertiefungen (24) oder Erhöhungen (18) versehen ist.

4. Stabilisierungsanordnung nach Anspruch 1 oder 2, **dadurch gekennzeichnet, dass** lediglich ein Teilbereich der Aussenseite (14) mit Vertiefungen (24) oder Erhöhungen (18) versehen ist.

5. Stabilisierungsanordnung nach einem der Ansprüche 1 bis 4, **dadurch gekennzeichnet, dass** er aus Titan, Stahl oder Kunststoff und insbesondere faserverstärktem Kunststoff hergestellt ist.

6. Stabilisierungsanordnung nach einem der Ansprüche 1 bis 5, **dadurch gekennzeichnet, dass** der Abstand zwischen zwei benachbarten Vertiefungen (24) oder Erhöhungen (18) grösser als 0,5 mm, vorzugsweise grösser als 0,7 mm und noch bevorzugter grösser als 0,9 mm ist.

7. Stabilisierungsanordnung nach einem der Ansprüche 1 bis 6, **dadurch gekennzeichnet, dass** der Abstand zwischen zwei benachbarten Vertiefungen (24) oder Erhöhungen (18) kleiner als 4,0 mm, vorzugsweise kleiner als 3,0 mm und noch bevorzugter kleiner als 0,7 mm ist.

8. Stabilisierungsanordnung nach Anspruch 1, **dadurch gekennzeichnet, dass** die Pedikelschraube (2) zwei oder mehr als zwei Erhöhungen (23) oder Vertiefungen (19) aufweist.

9. Stabilisierungsanordnung nach Anspruch 1 oder 8, **dadurch gekennzeichnet, dass** die Erhöhung (23) oder Vertiefung (19) der Pedikularschraube (2) korrespondierend zur Vertiefung (24) bzw. zur Erhöhung (18) des Verbindungsstabes (6, 6') ausgebildet ist.

## Claims

1. Stabilisation arrangement with
a connecting rod and
at least two clamping devices (3), which are each connectable to a vertebral body of a spine, and which each comprise a carrier (5) with an inner thread (22) or an outer thread (26) and are connected to a pedicle screw (2),
wherein the connecting rod comprises a first and a second end as well as an outer side (14) extending between these ends, to which outer side (14) in each case a clamping element (4) of said clamping devices (3) can be applied, wherein said outer side (14) of the connecting rod is provided with a plurality of concave depressions (24) or convex elevations (18) for forming a form-fit connection in circumferential direction and in longitudinal direction,
wherein both clamping devices (3) in each case comprise a passage opening (34), through which the connecting rod (6) can be pushed through and in which it can be fixed in a form-fit manner in longitudinal direction and in circumferential direction,
wherein the clamping element (4) of the clamping devices (3) in each case can be screwed into the inner thread (22) of the carrier (5) or onto the outer thread (26) of the carrier (5);
and wherein the clamping element (4) comprises at least one elevation (27) or at least one depression, which can be applied to the at least one concave depression (24) or to the at least one convex elevation (18) of the connecting rod for forming a form-fit connection, such that the connecting rod, when the clamping element (4) is tightened, is fixed in a form-fit manner in the clamping device both in the longitudinal direction and in the circumferential direction, and/or the pedicle screw (2) comprises an elevation (23) or a depression (19), which in each case can be applied to the at least one concave depression (24) or convex elevation (18) of the connecting rod (6) for forming a form-fit connection, such that the connecting rod, when the clamping element (4) is tightened, is fixed in a form-fit manner in the clamping device both in longitudinal direction an in circumferential direction.

2. Stabilisation arrangement according to claim 1, **characterized in that** it comprises an essentially round, oval, crescent-shaped or star-shaped cross-section.

3. Stabilisation arrangement according to claim 1 or 2, **characterized in that** essentially the entire outer side (14) is provided with depressions (24) or elevations (18).

4. Stabilisation arrangement according to claim 1 or 2, **characterized in that** only a portion of the outer side (14) is provided with depressions (24) or elevations (18).

5. Stabilisation arrangement according to one of claims 1 to 4, **characterized in that** it is produced from titan, steel or from plastic and in particular from fibre-reinforced plastic.

6. Stabilisation arrangement according to one of claims 1 to 5, **characterized in that** the distance between two neighbouring depressions (24) or elevations (18) is larger than 0.5 mm, preferably larger than 0.7 mm and more preferably larger than 0.9 mm.

7. Stabilisation arrangement according to one of claims 1 to 6, **characterized in that** the distance between two neighbouring depressions (24) or elevations (18) is smaller than 4.0 mm, preferably smaller than 3.0 mm and more preferably smaller than 0.7 mm.

8. Stabilisation arrangement according to claim 1, **characterized in that** the pedicle screw (2) comprises two or more than two elevations (23) or depressions (19).

9. Stabilisation arrangement according to claim 1 or 8, **characterized in that** the elevation (23) or depression (19) of the pedicle screw (2) is formed correspondingly to the depression (24) or to the elevation (18) of the connecting rod (6, 6').

## Revendications

1. Ensemble de stabilisation avec une tige de connexion et au moins deux dispositifs de serrage (3), lesquels peuvent respectivement être connectés à un corps de vertèbre d'une colonne vertébrale, et qui respectivement présentent un support (5) ayant un filetage femelle (22) ou filetage mâle (23) et qui sont connectés avec une vis pédiculaire (2),
où la tige de connexion présente une première et une deuxième extrémité ainsi qu'un côté extérieur (14) s'étendant entre ces extrémités, sur lesquelles un élément de serrage (4) desdits dispositifs de serrage (3) peut être appliqué, où ledit côté extérieur (14) de la tige de connexion est pourvu d'une pluralité d'évidements concaves (24) ou de surélévations convexes (18) pour former une connexion par complémentarité de forme en direction circonférentielle et en direction longitudinale,
où les deux dispositifs de serrage (3) présentent respectivement une ouverture de passage (34), par laquelle la tige de connexion (6) peut être coulissée à travers et dans laquelle elle peut être fixée par complémentarité de forme en direction circonférentielle et en direction longitudinale,
où l'élément de serrage (4) des dispositifs de serrage (3) peut respectivement être vissé dans le filetage femelle (22) du support (5), respectivement vissé sur le filetage mâle (23), du support (5),
et où l'élément de serrage (4) présente au moins une surélévation (27) ou au moins un évidement qui peut être appliqué(e) pour une connexion par complémentarité de forme à l'au moins un évidement concave (24) respectivement à l'au moins une surélévation convexe (18) de la tige de connexion, de sorte que lorsque l'élément de serrage (4) est serré, la tige de connexion est fixée par complémentarité de forme en direction longitudinale ainsi qu'en direction circonférentielle dans le dispositif de serrage, et/ou la vis pédiculaire (2) présente un évidement (19) ou une surélévation (23) qui peut respectivement être appliqué(e) pour former une connexion par complémentarité de forme à au moins un évidement concave (24) respectivement à au moins une surélévation convexe (18) de la tige de connexion (6), de sorte que lorsque l'élément de serrage (4) est serré, la tige de connexion est fixée par complémentarité de forme en direction longitudinale ainsi qu'en direction circonférentielle dans le dispositif de serrage.

2. Ensemble de stabilisation selon la revendication 1, **caractérisé en ce qu'**il présente une coupe transversale essentiellement ronde, ovale, en forme de croissant de lune ou en forme d'étoile.

3. Ensemble de stabilisation selon la revendication 1 ou 2, **caractérisé en ce que** essentiellement le côté extérieur entier (14) est pourvu d'évidements (24) ou de surélévations (18).

4. Ensemble de stabilisation selon la revendication 1 ou 2, **caractérisé en ce que** uniquement un domaine partiel du côté extérieur (14) est pourvu d'évidements (24) ou de surélévations (18).

5. Ensemble de stabilisation selon une des revendications 1 à 4, **caractérisé en ce qu'**il est d'élaboré en titane, en acier ou matière plastique et en particulier matière plastique renforcée avec des fibres.

6. Ensemble de stabilisation selon une des revendications 1 à 5, **caractérisé en ce que** l'espace entre deux évidements voisins (24) ou surélévations (18) est supérieur à 0.5 mm, préférablement supérieur à 0.7 mm et plus préférablement supérieur à 0.9 mm.

7. Ensemble de stabilisation selon une des revendications 1 à 6, **caractérisé en ce que** l'espace entre deux évidements voisins (24) ou surélévations (18) est inférieur à 4.0 mm, préférablement inférieur à 3.00 mm et plus préférablement inférieur à 0.7 mm.

8. Ensemble de stabilisation selon la revendication 1, **caractérisé en ce que** la vis pédiculaire (2) présente deux ou plus que deux surélévations (23) ou évidements (19).

9. Ensemble de stabilisation selon la revendication 1 ou 8, **caractérisé en ce que** la surélévation (23) ou l'évidement (19) de la vis pédiculaire (2) est formé(e) de manière correspondante à l'évidement (24), respectivement à la surélévation (18), de la tige de connexion (6, 6')
